(19) Europäisches Patentamt
European Patent Office
Office européen des brevets

(11) Publication number: **0 146 373**
**B1**

(12) **EUROPEAN PATENT SPECIFICATION**

(45) Date of publication of the patent specification: **05.11.86**

(21) Application number: **84308774.3**

(22) Date of filling: **14.12.84**

(51) Int. Cl.⁴: **C 07 C 51/29**, C 07 C 51/285, C 07 C 57/44, C 07 C 57/42, C 07 C 63/06, C 07 C 57/30, C 07 C 57/03, C 07 C 57/60, C 07 C 65/21, C 07 C 79/46, C 07 C 103/46

(54) **Process for oxidizing aldehydes to carboxylic acids.**

(30) Priority: **16.12.83 IT 2420283**

(43) Date of publication of application: **26.06.85 Bulletin 85/26**

(45) Publication of the grant of the patent: **05.11.86 Bulletin 86/45**

(84) Designated Contracting States: **BE CH DE FR GB LI NL**

(56) References cited: **GB-A-1 549 140**

(73) Proprietor: **Montedison S.p.A., 31, Foro Buonaparte, I-20121 Milan (IT)**

(72) Inventor: **Dalcanale, Enrico, 14, Via Visentin, I-28100 Novara (IT)**
Inventor: **Bottaccio, Giorgio, 16, Via Manin, Novara (IT)**
Inventor: **Campolmi, Stefano, 27, Via A. Costa, Novara (IT)**
Inventor: **Montanari, Fernando, 29, Via Muratori, I-20100 Milan (IT)**

(74) Representative: **Whalley, Kevin, Marks & Clerk 57/60 Lincoln's Inn Fields, London WC2A 3LS (GB)**

**EP 0 146 373 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convension).

LIBER, STOCKHOLM 1986

**Description**

This invention relates to a process for oxidizing aldehydes to carboxylic acids.

More particularly, the present invention relates to a method of preparing carboxylic acids from aldehydes in the presence of the couple $M(C10_2)_n/H_2O_2$, where M is an alkali metal or an alkaline-earth metal and n is an integer which may be 1 or 2, at a regulated pH, in aqueous-organic mixed solvents.

The acids obtained correspond to the general formula

R - COOH (I)

in which R is an aryl, aralkyl, aralkenyl, aralkynyl, heteroaryl, heteroarylalkyl, heteroarylalkenyl, heteroarylalkynyl, alkyl, alkenyl or alkynyl group, these groups preferably containing up to 20 carbon atoms and being optionally substituted by one or more substituents inert under the reaction conditions. Preferred heteroatoms which are present in group R of formula (I) are N, O and S.

The carboxylic acids of formula (I) and obtained according to the process of the present invention are interesting products usefully employable in a wide range of industrial applications.

In particular, the products of formula (I) are industrially utilizable as intermediates for the production of pharmaceutical products (α-aminoacids, etc.), for alimentary uses (synthetic sweeteners, etc.) and, more generally, as intermediates in the field of the so-called fine chemicals.

It is known to carry out the oxidation of aldehydes to carboxylic acids by using chlorites of formula $M(C10_2)_n$ in the presence of reagents, which are called scavengers and have the task of subtracting, by forming an addition product therewith, the hypochlorous acid HC10 which forms as a by-product during the oxidation; in fact, the hypochlorous acid may give rise to undesired secondary reactions, such as addition to double bonds possibly present in the molecule of the aldehyde to be oxidized, epoxidizing reactions, chlorination reactions etc.

Scavengers known and described for example in "Acta Chimica Scandinavica" 27 (1973) 888-890 and "Tetrahedron" vol. 37 (1981) 2091-2096 are sulphamic acid, polyphenols and 2-methyl-2-butene. These scavengers form addition compounds with hypochlorous acid which pollute the reaction product to be obtained, i.e. the carboxylic acid, and are difficult to separate from it.

It is an aim of the present invention to provide a process for oxidizing aldehydes to carboxylic acids by means of chlorites, avoiding the drawbacks mentioned hereinbefore.

Another aim of the present invention is that of providing a process for oxidizing aldehydes to carboxylic acids by means of chlorites, which is characterized by simple operative conditions, high yields of carboxylic acids and a high purity of such acids.

A further aim of the present invention is that of providing a scavenger of hypochlorous acid which does not give rise to undesired secondary reactions and provides by-products which are removable without difficulties from the reaction product.

The present invention provides a process for oxidizing aromatic, heteroaromatic and aliphatic aldehydes having the general formula:

R-CHO

wherein R is as defined above, to the corresponding acids of general formula:

R-COOH

wherein R is as defined above, by means of an alkali metal or alkaline-earth metal chlorite, in an aqueous-organic medium, characterized in that the reaction is conducted in the presence of hydrogen peroxide.

Thus by means of the process of the present invention there may be achieved the oxidation of aldehydes of general formula:

R - CHO (II) to carboxylic acids R-COOH (I), by using as an oxidant a salt of the type

$M(C10_2)_n$ (III)

wherein M and n have the same meanings as defined above, and, as a scavenger, hydrogen peroxide, in a mixed aqueous-organic solvent, and preferably at a regulated pH ranging from 1 to 6 and preferably at a temperature of from 0° to 100° C, according to the following reaction scheme:

$$R - CHO + C10_2^- \xrightarrow{H^+} R - COOH + HC10$$

In fact it has been found that hydrogen peroxide, under the employed reaction conditions, acts as a reducing agent towards the hypochlorous acid which has formed during the reaction:

$HOCl + H_2O_2 \rightarrow HCl + H_2O + O_2\uparrow$

The products which form in consquence of this reaction are all easily removable from the reaction product and do not react with the organic substrate, i.e. with the aldehyde or the carboxylic acid. Furthermore, the use of hydrogen peroxide prevents the formation of chlorine dioxide which is due to the reaction between chlorite and hypochlorous acid, which is a highly toxic gas and causes undesired secondary reactions.

The aldehydes utilizable according to the process of the present invention are of the aromatic, araliphatic,

heteroaromatic, heteroarylaliphatic and aliphatic types. The aromatic and heteroaromatic nuclei present in the aldehydes may consist of one or more rings, either condensed or not, and contain up to 20 carbon atoms. By way of example only there may be mentioned phenyl, naphthyl, diphenyl, furyl, thienyl, pyridyl etc. The heteroatom may be N, S, O.

The aliphatic groups which are present in the aldehydes may be of the linear or ramified type, either saturated or unsaturated and contain in aggregate up to 20 carbon atoms; alkyl, alkenyl and alkynyl groups may be present.

Both the aromatic nuclei and the heteroaromatic nuclei may contain substituents which are inert under the reaction conditions, such as halogen-, carboxy-, ester-, ether-, amido-, nitro-, alkyl-, alkenyl-, and alkynyl-groups.

The aliphatic groups also may be substituted by substituents inert under the reaction conditions, such as halogen-, carboxy-, ester-, ether-, amido-, and nitro-groups.

Examples of aldehydes which are oxidizable to the corresponding acids according to the process of the present invention are as follows:

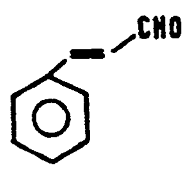
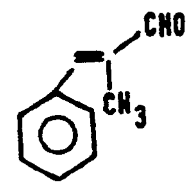
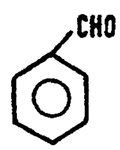
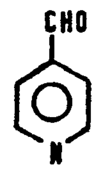
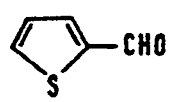
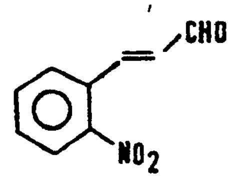
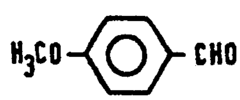
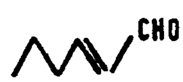
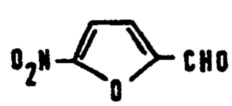
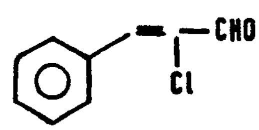
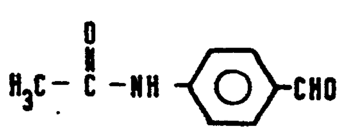
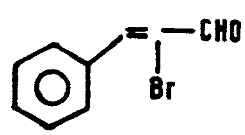
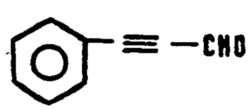
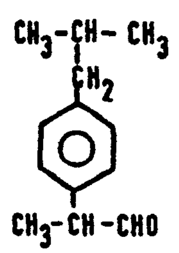

The oxidizing system consists of salts of the type $M(C10_2)_n$, preferably of sodium and of potassium.

The solvents are composed of water and of organic solvents, either miscible or non-miscible with the aqueous phase. The solvents can be suitably alcohols, especially aliphatic alcohols, ethers, cyclic or acyclic, and nitriles, etc. Tetrahydrofuran, acetonitrile, methanol, ethanol, iso-butanol, and ter.butanol are preferably utilized.

The reaction temperatures preferably range from 0° to 100°C, more preferably from 10°C to 40°C; the reaction times preferably vary from 2 to 8 hours approximately, more preferably from 2 to 6 hours approximately; the substrate/$M(C10_2)_n/H_2O_2$ molar ratios, i.e. aldehyde/chlorite/hydrogen peroxide molar ratios, preferably range from 1:1:1 to 1:5:5, more preferably from 1:1:1 to 1:2:5; and the pH is preferably from 1 to 6, more preferably from 3 to 6.

When the reaction is operated outside the preferred conditions indicated, the desired oxidation of the aldehyde group to acid either does not occur, or it occurs in an unsatisfactory manner with low selectivities.

The separation of the reaction product is carried out according to conventional techniques.

For example, an extraction is carried out with ether, and the ether extract is extracted in turn with aqueous sodium carbonate; the aqueous phase is then acidified and extracted with ether; from the organic phase anhydrified on sodium sulphate and evaporated, the product (1) is obtained.

As an alternative, for products insoluble in water, the organic phase of the mixture is evaporated under vacuum, and the product (I) precipitates and is separated by filtration.

The remaining aqueous phase is treated with sodium sulphite to remove unreacted residues, if any, of $M(C10_2)_n$, HC10 and $H_2O_2$.

The process according to the invention may be suitably conducted as follows.

Into a thermoregulated reactor equipped with a magnetic stirrer, a thermometer, a dropping funnel and a gas outlet connected with a bubbler (bubbling scrubber), there are introduced the solvent, the aldehyde (II), an aqueous solution buffered with monobasic sodium phosphate or other buffer substance conventionally employed, at a pH equal to about 4.3, and hydrogen peroxide. An aqueous solution of $M(C10_2)_n$ (III) is added dropwise, in about 2 hours and at room temperature, into the flask containing said mixture. The whole is then allowed to react, always at room temperature, for a prefixed time ranging from 2 to 5 hours, until complete disappearance of the aldehyde occurs, which is ascertained by gas-chromatography.

Product (I) is separated as mentioned hereinbefore.

Due to the simple operative conditions and to the low cost of the reagents, the process is particularly advantageous.

Another advantage consists in the high yields and purity of the products obtained and in the complete conversion of the aldehyde (II), these factors being of particular interest in view of the applications of the products in the field of fine chemicals.

The invention will be further described with reference to the following illustrative Examples.

**Example 1**

Into a 250 cc flask, equipped with a magnetic stirrer, a reflux cooler connected with a bubbler, a thermometer and a dropping funnel, there were added in order:

50 cc of acetonitrile, 6.30 cc (5.0. $10^{-2}$ moles) of cinnamaldehyde at 99%, 1.6 g of $NaH_2PO_4$ dissolved in 20 cc of $H_2O$, and 5.00 cc of hydrogen peroxide at 35% (5.2. $10^{2}$ moles) (oxidimetric titre).

The temperature was maintained at about 30°C by means of a water bath. To the solution there were added 8.00 g (7.0. $10^{-2}$ moles) of $NaC10_2$ at 79% (iodometric titre) dissolved in 70 cc of $H_2O$.

The addition was carried out in a time varying from 2 to 4 hours, while the pH varied from 5 to 3.

After the addition, the mixture was allowed to react for about 1 hour, then the product was separated by a conventional method, such as extraction with solvent, precipitation, etc. 7.0 g of cinnamic acid as a crystalline white product, with a yield of 95% calculated on the basis of the aldehyde at 99%, were obtained.

**Example 2**

By operating as in example 1, but using 50 cc of iso-butanol as a solvent and employing 8.00 g (7.0. $10^{-2}$ moles) of $NaC10_2$ at 79% dissolved in 23 cc of $H_2O$, 6.5 g of cinnamic acid with a yield of 89% calculated on the basis of the aldehyde at 99% were obtained.

**Example 3 to 5**

By operating according to example 1 and using different solvents, the following results were obtained from the oxidation of cinnamaldehyde to cinnamic acid:

| Example | Solvent | Yield % |
|---|---|---|
| | cc 50 | |
| 3 | $CH_3OH$ | 89% |
| 4 | $CH_3CH_2-OH$ | 91% |
| 5 | $(CH_3)_3C-OH$ | 95% |

**Example 6**

By operating as in example 3, using 25 cc of methanol instead of 50 cc, 6.3 g of cinnamic acid with a yield of 86% were obtained.

**Example 7 to 19**

By operating as in example 1 and using different aromatic, heteroaromatic, and aliphatic aldehydes substituted by inert groups, the following results were obtained:

FIG4/27

| Example No. | Aldehyde | Yield % | Solvent |
|---|---|---|---|
| 7 | 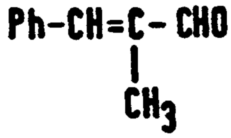 | 93% | tetrahydrofuran |
| 8 | Ph – CHO | 93% | $CH_3OH$ |
| 9 | 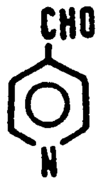 | 100% | $CH_3CN$ |
| 10 | 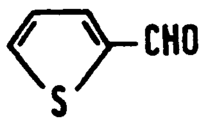 | 94% | $CH_3CN$ |
| 11 | 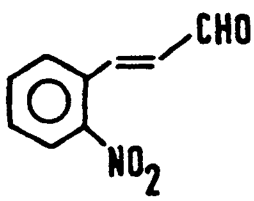 | 98% | $CH_3CN$ |
| 12 | 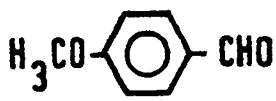 | 86% | $CH_3CN$ |
| 13 | 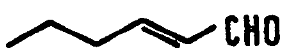 | 88% | $CH_3CN$ |
| 14 | 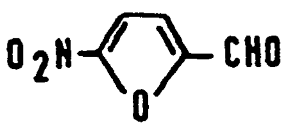 | 84% | $CH_3CN$ (1) |
| 15 | 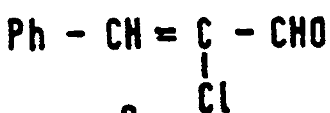 | 93% | $CH_3CN$ (2) |
| 16 | 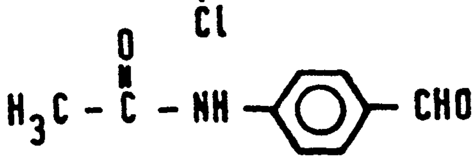 | 96% | $CH_3CN+CH_3OH$ |
| 17 | 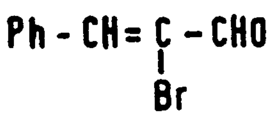 | 94% | $CH_3CN$ |
| 18 | Ph – C≡C – CHO | 74% | $CH_3CN$ (1) |
| 19 | p-isobutyl-2-phenylpropionaldehyde | 97% | isobutanol |

(1) In respect of example 1, there was used an amount of $H_2O_2$ with a molar ratio to the aldehyde of 5:1 and a pH of about 2.

(2) In respect of example 1, there was used a higher amount of $NaClO_2$ and of $H_2O_2$: 9.00 g of $NaClO_2$ and 6.00 cc of $H_2O_2$.

**Example 20**

(comparative test)

Into a 3-neck, 250 cc flask equipped with a magnetic stirrer, a reflux cooler connected with a bubbler, a thermometer and a dropping funnel, there were introduced in the following order:

50 cc of ter. butanol, 6.3 cc of cinnamaldehyde at 99% ($5.10^{-2}$ moles), and 6.3 g of $NH_2SO_3H$ ($6.5. 10^{-2}$ moles) in 60 cc of $H_2O$. In a beaker, a solution containing 7.0 g of $NaClO_2$ at 79% ($6.1. 10^{-2}$ moles) in 40 cc of $H_2O$ was prepared; this solution was slowly dropped into the reaction flask in a time of 1 hour.

After the addition, the mixture was allowed to react for about 1 hour. A mixture was obtained containing 4.4 g of cinnamic acid corresponding to a 60% yield calculated on the basis of the 99% aldehyde, 1.64 g of cinnamonitrile (by-product obtained in the presence of the scavenger) and 0.1 g of terbutyl cinnamate.

**Claims**

1. A process for oxidizing aromatic, heteroaromatic and aliphatic aldehydes having the general formula:

R - CHO

wherein R is selected from aryl, aralkyl, aralkenyl, aralkynyl, heteroaryl, heteroarylalkyl, heteroarylalkenyl, heteroarylalkynyl, alkyl, alkenyl and alkynyl groups, optionally substituted by one or more substituents which are inert under the reaction conditions, to the corresponding acids of general formula:

R - COOH

wherein R is as defined above, by means of an alkali metal or alkaline-earth metal chlorite, in an aqueous-organic medium, characterized in that the reaction is conducted in the presence of hydrogen peroxide.

2. A process as claimed in claim 1, characterized in that the reaction is conducted at a temperature from 0°C to 100°C and at a pH from 1 to 6.

3. A process as claimed in claim 2, characterized in that the reaction is conducted at a temperature from 10° to 40°C and at a pH from 3 to 6.

4. A process as claimed in any of claims 1 to 3, characterized in that the aldehyde/chlorite/hydrogen peroxide molar ratio is from 1:1:1 to 1:5:5.

5. A process as claimed in claim 4, characterized in that the aldehyde/chlorite/hydrogen peroxide molar ratio is from 1:1:1 to 1:2:5.

6. A process as claimed in any of claims 1 to 5, characterized in that the chlorite is sodium or potassium chlorite.

7. A process as claimed in any of claims 1 to 6, characterized in that the reaction medium is a mixture of water and of a solvent selected from aliphatic alcohols, cyclic ethers, acyclic ethers, and nitriles.

8. A process as claimed in claim 7, characterized in that the said solvent is selected from methanol, ethanol, iso-butanol, ter.butanol, tetrahydrofuran and acetonitrile.

9. A process as claimed in any of claims 1 to 8, characterized in that the aldehyde R-CHO is selected from cinnamaldehyde, α-methylcinnamaldehyde, benzaldehyde, 4-pyridinaldehyde, 2-thiophenaldehyde, o.nitrocinnamaldehyde, p.methoxybenzaldehyde, 3-propylacrolein, 4-nitro-2-furaldehyde, α-chlorocinnamaldehyde, p.acetamidobenza ldehyde, α-bromocinnamaldehyde, β-phenylpropargylaldehyde, and p-isobutyl-2-phenylpropionaldehyde.

**Patentansprüche**

1.- Verfahren zur Oxidation von aromatischen, heteroaromatischen und aliphatischen Aldehyden mit der allgemeinen Formel

R - CHO

worin R ausgewählt ist aus Aryl-, Aralkyl-, Aralkenyl-, Aralkinyl-, Heteroaryl-, Heteroarylalkyl-, Heteroarylalkenyl-, Heteroarylalkinyl-, Alkyl-, Alkenyl- und Alkinylgruppen, gegebenenfalls substituiert durch einen oder mehrere, unter den Reaktionsbedingungen inerte Substituenten, zu den entsprechenden Säuren der allgemeinen Formel

R - COOH

worin R wie oben definiert ist, mittels eines Alkalimetalloder Erdalkalimetallchlorits in einem wässrigorganischen Medium, dadurch gekennzeichnet, daß die Reaktion in Anwesenheit von Wasserstoffperoxid

durchgeführt wird.

2.- Verfahren nach Anspruch 1, dadurch gekennzeichnet, daß die Reaktion bei einer Temperatur von 0 bis 100° C und einem pH von 1 bis 6 durchgeführt wird.

3.- Verfahren nach Anspruch 2, dadurch gekennzeichnet, daß die Reaktion bei einer Temperatur von 10 bis 40° C und einem pH von 3 bis 6 durchgeführt wird.

4.- Verfahren nach einem der Ansprüche 1 bis 3, dadurch gekennzeichnet, daß das molare Verhältnis von Aldehyd/ Chlorit/Wasserstoffperoxid von 1:1:1 bis 1:5:5 beträgt.

5.- Verfahren nach Anspruch 4, dadurch gekennzeichnet, daß das molare Verhältnis von Aldehyd/Chlorit/Wasserstoffperoxid von 1:1:1 bis 1:2:5 beträgt.

6.- Verfahren nach einem der Ansprüche 1 bis 5, dadurch gekennzeichnet, daß das Chlorit Natrium- oder Kaliumchlorit ist.

7.- Verfahren nach einem der Ansprüche 1 bis 6, dadurch gekennzeichnet, daß das Reaktionsmedium eine Mischung aus Wasser und einem Lösungsmittel, ausgewählt aus aliphatischen Alkoholen, cyclischen Ethern, acyclischen Ether und Nitrilen, ist.

8.- Verfahren nach Anspruch 7, dadurch gekennzeichnet, daß das Lösungsmittel ausgewählt ist von Methanol, Ethanol, i-Butanol, tert-Butanol, Tetrahydrofuran und Acetonitril.

9.- Verfahren nacn einem der Ansprüche 1 bis 8, dadurch gekennzeichnet, daß der Aldehyd R-CHO ausgewählt ist von Zimtaldehyd, α-Methylzimtaldehyd, Benzaldehyd, 4-Pyridin-aldehyd, 2-Thiophenaldehyd, o-Nitrozimtaldehyd, p-Methoybenzaldehyd, 3-Propylacrolein, 4-Nitro-2-furaldehyd, α-Chlorzimtaldehyd, p-Acetamindobenzaldehyd, α-Bromzimtaldenyd, β-Phenylpropargylaldehyd und p-Isobutyl-2-pnenylpropionaldehyd.

**Revendications**

1.- Un procédé d'oxydation d'aldéhydes aromatiques, hétéroaromatiques et aliphatiques de formule générale:

R - CHO

dans laquelle:

R est choisi parmi les groupes aryle, aralkyle, aralkényle, aralkynyle, hétéroaryle, hétéroarylalkyle, hétéroarylalkényle, hétéroarylalkynyle, alkyle, alkényle et alkynyle, éventuellement substitués par un ou plusieurs substituants inertes dans les conditions de la réaction, en les acides correspondants de formule générale:

R - COOH

dans laquelle R est tel que défini ci-dessus, au moyen d'un chlorite de métal alcalin ou de métal alcalino-terreux, en milieu aqueux-organique, caractérisé en ce que l'on conduit la réaction en présence de peroxyde d'hydrogène.

2.- Un procédé selon la revendication 1, caractérisé en ce que l'on conduit la réaction à une température de 0° à 100°C et à un pH de 1 à 6.

3.- Un procédé selon la revendication 2, caractérisé en ce que l'on conduit la réaction à une température de 10° à 40°C et à un pH de 3 à 6.

4.- Un procédé selon l'une quelconque des revendications 1 à 3, caractérisé en ce que le rapport molaire aldéhyde/chlorite/peroxyde d'hydrogène est compris entre 1/1/1 et 1/5/5.

5.- Un procédé selon la revendication 4, caractérisé en ce que le rapport molaire aldéhyde/chlorite/peroxyde d'oxygène est compris entre 1/1/1 et 1/2/5.

6.- Un procédé selon l'une quelconque des revendications 1 à 5, caractérisé en ce que le chlorite est du chlorite de sodium ou du chlorite de potassium.

7.- Un procédé selon l'une quelconque des revendications 1 à 6, caractérisé en ce que le milieu réactionnel est un mélange d'eau et de solvant choisi parmi les alcools aliphatiques, les éthers cycliques, les éthers acycliques et les nitriles.

8.- Un procédé selon la revendication 7, caractérisé en ce que ce solvant est choisi parmi le méthanol, l'éthanol, l'isobutanol, le tertiobutanol, le tétrahydrofuranne et l'acétonitrile.

9.- Un procédé selon l'une quelconque des revendications 1 à 8, caractérisé en ce que l'aldéhyde R-CHO est choisi parmi les cinnamaldéhyde, a-méthylcinnamaldéhyde, benzaldéhyde, 4-pyridinaldéhyde, 2-thiophénaldéhyde, o.nitrocinnamaldéhyde, p.méthoxybenzaldéhyde, 3-propylacroléine, 4-nitro-2-furaldéhyde, α-chlorocinnamaldéhyde, p.acétamidobenzaldéhyde, α-bromocinnamaldéhyde, β-phénylpropargylaldéhyde, et p-isobutyl-2-phényl-propionaldéhyde.